# EUROPÄISCHE PATENTSCHRIFT

(11) **EP 1 154 766 B1**
(45) Veröffentlichungstag und Bekanntmachung des Hinweises auf die Patenterteilung: **03.01.2007**
(21) Anmeldenummer: 00904922.2
(22) Anmeldetag: 17.01.2000
(51) Int. Cl.: A61K 31/192, A61P 29/00

(54) **VERWENDUNG VON R-ARYLPROPIONSÄUREN ZUR HERSTELLUNG VON ARZNEIMITTELN, ZUR BEHANDLUNG VON ERKRANKUNGEN BEI MENSCH UND TIER, WELCHE DURCH DIE HEMMUNG DER AKTIVIERUNG VON NF-KAPPA B THERAPEUTISCH BEEINFLUSST WERDEN KÖNNEN**
USE OF R-ARYL PROPIONIC ACIDS FOR PRODUCING MEDICAMENTS TO TREAT DISEASES IN HUMANS AND ANIMALS, WHEREBY SAID DISEASES CAN BE THERAPEUTICALLY INFLUENCED BY INHIBITING THE ACTIVATION OF NF-KAPPA B
UTILISATION D'ACIDES R-ARYL-PROPIONIQUES POUR LA PRODUCTION DE MEDICAMENTS, POUR LE TRAITEMENT DE MALADIES TOUCHANT L'HOMME ET L'ANIMAL ET SUR LESQUELLES L'INHIBITION DE L'ACTIVATION DU FACTEUR DE TRANSCRIPTION NF-KAPPA B PEUT EXERCER UNE INFLUENCE D'UN POINT DE VUE THERAPEUTIQUE

(30) Priorität: 24.02.1999 DE 19907895
(43) Veröffentlichungstag der Anmeldung: 21.11.2001
(73) Patentinhaber: PAZ Arzneimittel- Entwicklungsgesellschaft mbH, 65933 Frankfurt am Main (DE)
(72) Erfinder: GEISSLINGER, Gerd, D-65812 Bad Soden (DE); BRUNE, Kay, 91080 Marloffstein (DE)
(74) Vertreter: Wagner, Jutta
(86) Internationale Anmeldenummer: PCT/EP2000/000323
(87) Internationale Veröffentlichungsnummer: WO 2000/050019

(56) Entgegenhaltungen:
- WO-A-00/13684
- WO-A-98/09603
- WO-A-98/47502
- DE-C1- 4 319 438
- US-A- 5 200 198
- N. SCEUREN : "Modulation of transcription factors by nonsteroidal anti-inflammatory drugs" NAUNYN-SCHMIEDEBERG'S ARCH. PHARMACOL., Bd. 354, Nr. 4 suppl. 1, 1996, Seite R5 XP000938437
- N. SCHEUREN: "Enantiomers of the nonsteroidal anti-inflammatory drug ibuprofen are potent and specific inhibitors of transcription factor NF-kappa,beta" NAUNYN SCHMIEDEBERG'S ARCH PHARMACOL., Bd. 357, Nr. 4 supl., 1998, Seite R16 XP000938436
- N. SCHEUREN: "Modulation of transcription factor NF-kapa,beta by enantiomers of the nonsteroidal drug ibuprofen " BR. J. PHARMACOL., Bd. 123, Nr. 4, 1998, Seiten 645-652, XP000938435
- N. SCEUREN: "Weak inhibitors of cyclooxygenases may exert their antinociceptive effect by modulation of transcription factors" ADV. EXP. MED. BIOL., Bd. 433, 1997, Seiten 51-54, XP000938438

## Beschreibung

Gegenstand der vorliegenden Erfindung ist die Verwendung von R-Arylpropionsäuren zur Herstellung von Arzneimitteln, zur Behandlung von Erkrankungen bei Mensch und Tier, welche durch die Hemmung der Aktivierung von NF-κB therapeutisch beeinflußt werden können.

Arylpropionsäuren und deren Derivate werden seit langem als nichtsteroidale antiinflammatorisch und analgetisch wirksame Arzneimittel eingesetzt. Bekannte Vertreter dieser Wirkstoffgruppe sind Ibuprofen, Flurbiprofen, Ketoprofen, Naproxen, Tiaprofensäure und Fenoprofen [Propionic acid derivatives; Goodman & Gilman's, The pharmacological basis of therapeutics, Chapter 27, p. 637 (Ninth Edition, 1996)].

Aufgrund der Molekülstruktur mit einem asymmetrischen C-Atom sind Arylpropionsäuren und deren Derivate chiral, kommen also als R- und S-enantiomere Form vor. Normalerweise fallen bei der chemischen Synthese diese Wirkstoffe als Racemat an. Bis auf S-Naproxen [Williams: Enantiomers in arthritic disorders; Pharmac. Ther., Vol. 46, pp. 273-295 (1990); Evans: Enantioselective pharmacodynamics and pharmacokinetics of chiral non-steroidal anti-inflammatory drugs. Eur J Clin Pharmacol 42: 237-256 (1992)] und neuerdings Dexibuprofen [Symposium: Update on S(+)-ibuprofen; Going/Kitzbühl 2 bis 4. Februar 1996] und Dexketoprofen [Scrip No. 1831 June 22^{nd} 1993 p. 7; Scrip No 2144 July 9^{th} 1996 p.16] werden diese Wirkstoffe in Arzneimitteln bislang als Racemate eingesetzt.

Die therapeutisch erwünschte entzündungshemmende und schmerzlindernde Wirkung der Arylpropionsäuren und deren Derivate wird im wesentlichen der Hemmung der Prostaglandinbiosynthese zugeschrieben [Vane and Botting: Overview - mechanism of action of antiinflammatory drugs. In: Improved non-steroidal antiinflammatory drugs - COX-2 enzyme inhibitors, S. 1-27, Lancester: Kluwer Academic Publishers (1996)]. Diese erfolgt über die Hemmung der bei der Bildung von Prostaglandinen beteiligten Enzyme Cyclooxygenase 1 und 2 (COX-1 und COX-2 bzw. PGHS-1 und PGHS-2). Durch die reduzierte Bildung von Prostaglandinen werden die mit diesen Entzündungsmediatoren im Zusammenhang stehenden Entzündungssymptome wie Schmerz, Schwellung, Rötung, Ödembildung, Erwärmung und Funktionseinschränkung abgeschwächt. Die Hemmung der Prostaglandinbiosynthese wird als gemeinsames Merkmal des Mechanismus der antiinflammatorischen und der analgetischen Wirkung angenommen. Die therapeutisch erwünschte Hemmung der Prostaglandinproduktion im erkrankten Zielgewebe führt in anderen Organsystemen, die auf das Vorhandensein bestimmter Prostaglandinkonzentrationen angewiesen sind, zu unerwünschten Arzneimittel-wirkungen. Insbesondere betroffen von den unerwünschten Wirkungen sind der Magen-Darm-Trakt, die Nieren, die Lungen und die Blutplättchen.

Es ist bekannt, daß in Bezug auf die Prostaglandinsynthesehemmung wesentliche Unterschiede zwischen den enantiomeren Formen der Arylpropionsäuren bestehen [Williams (s.o.); Evans (s.o.); Brooks and Day: New nonsteroidal anti-inflammatory drugs, Birkhauser Verlag, Basel, S. 119-126 (1985)]. Während alle S-Enantiomere dieser Substanzen eine ausgeprägte Prostaglandinsynthesehemmung aufweisen, wird diese bei den R-Enantiomeren im therapeutisch relevanten Konzentrationsbereich nicht gefunden. Folglich werden in therapeutischen Konzentrationen den R-Arylpropionsäuren und deren Derivaten weder die erwünschten, noch die unerwünschten Arzneimittelwirkungen zugeschrieben, die mit der Inhibition der Prostaglandinproduktion im Zusammenhang stehen. Unabhängig vom Fehlen dieser wirkungsmechanismusspezifischen unerwünschten Wirkungen können die R-Enantiomere dieser Wirkstoffklasse substanzspezifische unerwünschte Wirkungen aufweisen.

Wegen der bisherigen therapeutischen und wirtschaftlichen Bedeutung der als Racemat eingesetzten Arylpropionsäuren wird versucht, die Sinnhaftigkeit der Anwendung der racemischen Wirkstoffe zu begründen. Im Falle von Ibuprofen wird die Anwendung des Racemates im wesentlichen damit begründet, daß im menschlichen oder tierischen Organismus eine mehr oder weniger ausgeprägte Inversion von R-Ibuprofen zu S-Ibuprofen stattfindet [Caldwell et al.: The metabolic chiral inversion and dispositional enantioselectivity of the 2-arylpropionic acids and their biological consequences; Biochemical Pharmacology, Vol. 37, No. 1, pp. 105-114 (1988)], so daß auch ein Teil der R-Form nach Inversion zur S-Form als Prostaglandinsynthesehemmer wirksam werden kann. Außerdem wird für R-Ibuprofen eine Hemmung der polymorphkernigen Leukozyten in vitro beschrieben, die sich bei entzündlichen Erkrankungen als vorteilhaft erweisen könnte [Villanueva et al.: Equipotent inhibition by R(-), S(+)- and racemic ibuprofen of human polymorphonuclear cell function in vitro; Br. J. clin. Pharmac., 35, 235-242 (1993)]. Die therapeutische Relevanz dieses Mechanismus bei Anwendung von racemischem Ibuprofen konnte jedoch nicht gezeigt werden. Für R-Flurbiprofen ist die Inversion zu vernachlässigen.

Die Tatsache, daß die therapeutische Wirkung der Arylpropionsäuren im wesentlichen der Pröstaglandinsynthesehemmung zugeschrieben wird, hat zu der Erkenntnis geführt, daß die Anwendung der reinen S-Enantiomere, gegebenenfalls der racemischen Verbindungen, jedoch nicht der reinen R-Enantiomere sinnvoll sei. Erst mit der überraschenden Entdeckung, daß R-Flurbiprofen einen antinozizeptiven Effekt aufweist, der nicht mit der Hemmung der peripheren Prostaglandinbiosynthese im Zusammenhang steht, wurde die Entwicklung von Arzneimitteln auf der Basis von R-Flurbiprofen [DE 40 28 906 C2; EP 0 607 128 B1; USA 5,206,029 und 5,200,198] als Schmerzmittel ohne entzündungshemmende Wirkkomponente eingeleitet. Später wurde auch für R-Ketoprofen eine schmerzlindernde Wirkung beschrieben [DE 43 19 438 C1; WO 93/17667].

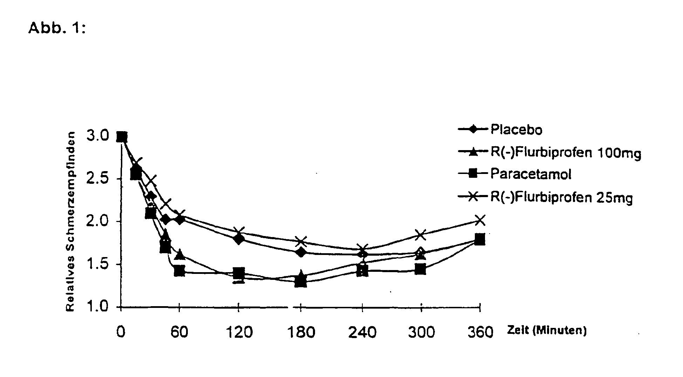

Neuere Publikationen bestätigen den antinozizeptiven Effekt von R-Flurbiprofen [Geisslinger, Schaible: New insights into the site and mode of antinociceptive action of flurbiprofen enantiomers, J Clin Pharmacol, 36, 513-520 (1996); Buritova, Besson: Peripheral and/or central effects of racemic-, S(+)- and R(-)-flurbiprofen on inflammatory nociceptive processes: a c-Fos protein study in the rat spinal cord; British J. Pharmacolocy, 125, 87-101 (1998)] In klinischen Studien an Patienten konnte die schmerzlindernde Wirkung von R-Flurbiprofen **[Abb. 1]** und R-Ketoprofen [Cooper et al.: Analgesic efficacy and safety of R-Ketoprofen in postoperative dental pain; J Clin Pharmacol, 38, 11 S-18S (1998)] nachgewiesen werden.

### Abb. 1: Placebo-kontrollierte Doppelblind-Studie an 180 Frauen mit akutem Postepisiotomie-Schmerz (Mittelwertkurven)

Die hospitalisierten Patienten wurden in drei Medikationsgruppen mit je 50 Patienten und einer Placebogruppe (30 Patienten) randomisiert. Jede Patientin erhielt innerhalb 48 Stunden nach der ansonsten normal verlaufenden Entbindung eine Einzeldosis der zu untersuchenden Studienmedikation (25 mg R(-)Flurbiprofen oder 100 mg R(-)Flurbiprofen oder 1000 mg Paracetamol) oder ein Placebo oral verabreicht. Kurz vor der oralen Gabe der Testpräparate bzw. des Placebos und zu genau festgelegten Untersuchungszeitpunkten (15, 30, 45, 60, 120, 180, 240, 300 und 360 Minuten) wurden die Patienten bezüglich ihres Schmerzempfindens befragt. Die Wirksamkeit der einzelnen Präparate wurde anhand einer Schmerzempfindungs-Skala (0 = keine, 1 = mild, 2 = moderat, 3 = stark) beurteilt. Die zeitlichen Verläufe sind in den in Abbildung 1 wiedergegebenen Mittelwertkurven der einzelnen Patientengruppen zusammengefaßt.

Tierexperimentelle Studien belegen, daß die Wirkung von R-Flurbiprofen über eine entzündungshemmende und eine antinozizeptive Wirkung am zentralen Nervensystem erklärt werden kann [Buritova (s.o.); Neugebauer et al.: Antinociceptive effects of R(-)- and S(+)-flurbiprofen on rat spinal dorsal horn neurons rendered hyperexcitable by an acute knee joint inflammation; J.Pharmacol Exp Ther, 275, 618-628 (1995)]. Die bekannte periphere entzündungshemmende und antinozizeptive Wirkung von Flurbiprofen konnte dagegen ausschließlich beim S-Enantiomeren gefunden werden [Buritova (s.o.) und Neugebauer (s.o.)]. Nach dem derzeitigen Stand des Wissens ergibt sich daraus die bedeutende Konsequenz, daß zur optimalen Behandlung der peripheren entzündlichen Erkrankungen S-Arylpropionsäuren als Mittel der Wahl eingesetzt werden sollten. Zur Verringerung der mit der Prostaglandinsynthesehemmung zusammen-hängenden unerwünschten Wirkungen auf den Magen-Darm-Trakt etc. sollte z.B. S-Flurbiprofen nicht oral eingenommen werden, sondern lokal auf die entzündete bzw. schmerzende Stelle appliziert werden. R-Flurbiprofen sollte jedoch wegen der zentralen Wirkung systemisch [Buritova (s.o.)], z.B. oral, intramuskulär oder intravenös appliziert werden.

Entgegen dieser neuesten Erkenntnis zur praktisch ausschließlich zentralen Wirkung von R-Flurbiprofen wurde nun erfindungsgemäß überraschend gefunden, daß R-Flurbiprofen in bestimmten Konzentrationen ein potenter und spezifischer Hemmer der Aktivierung des nukleären Transkriptionsfaktors NF-κB ist. NF-κB ist ein ubiquitärer Transkriptionsfaktor, der in Zellen bei Immun- und Entzündungsreaktionen sowie bei der Expression von Cytokinen, Chemokinen, Zelladhäsionsmolekülen, Wachstumsfaktoren, Immunrezeptoren, akute Phase Proteinen, diversen Enzymen und anderen Transkriptionsfaktoren eine zentrale Rolle einnimmt [Lee, Burckart: Nuclear factor kappa B: Important transcritpion factor and therapeutic target, J.Clin.Pharm., 38, 981-993 (1998)].

Die NF-κB-Aktivierung kann auf verschiedenen Stufen der Aktivierungskaskade durch verschiedene Wirkstoffe inhibiert werden. So inhibieren Glucocorticoide NF-κB durch direkte Assoziation oder durch Verstärkung der Expression. Cyclosporine und Tacrolimus verhindern die NF-κB-Aktivierung durch Inhibierung der Calcineurinwirkung der Phosphatase, die indirekt den I-κB-Abbau induziert. Deoxyspargualin inhibiert NF-κB durch Blockade seiner Kernverlagerung. Aspirin und Salicylate inhibieren vorgelagerte Ereignisse, welche die I-κB Phosphorylierung induzieren. Tepoxalin und Antioxidantien inhibieren die NF-κB-Aktivierung durch Veränderung des Redox-Zustandes der Zelle. Weitere Recherchen sind notwendig um spezifische Inhibitoren zur Behandlung von Krankheiten, die durch NF-κB beinflußt werden, zu entwickeln [Lee, Burckart: Nuclear factor kappa B: Important transcritpion factor and therapeutic target, J.Clin.Pharm., 38, 981-993 (1998)].

Es ist bekannt, daß R-Ibuprofen und S-Ibuprofen die Aktivierung des Transkriptionsfaktors NF-κB hemmen, was auf eine Wirkung des im Körper gebildeten CoA Thioester zurückgeführt wird. [N. Scheuren et al. "Modulation of transcription factors by nonsteroidal anti-inflammatory drugs", Naunyn-Schmiedeberg's Arch. Pharmacol., Bd. 354, Nr. 4 suppl. 1, 1996; N. Scheuren et al. "Enantiomers of the nonsteroidal anti-inflammatory drug ibuprofen are potent and specific inhibitors of transcription factor NF-kappa, beta", Naunyn-Schmiedeberg's Arch. Pharmacol., Bd. 357, Nr. 4 suppl., 1998; N. Scheuren et al. "Modulation of transcription factor NF-Kappa, beta by enantiomers of the nonsteroidal drug ibuprofen", Br. J. Pharmacol., Bd. 123, Nr. 4, 1998; N. Scheuren et al. "Weak inhibitors of cyclooxygenases may exert theit antinociceptive effects of modulation of transcription factors", Adv. Exp. Med. Biol., Bd. 433, 1997].

Die WO 98/47502 schlägt CoA Thioester von Arylpropionsäuren, Arylessigsäuren oder Acetylsalicylaten sowie R-Ibuprofen oder Racemate von Ibuprofen mit bis zu 49 % S-Ibuprofen als schmerz- und/oder entzündungshemmende Arzneimittel vor.

Die Erfindung hat sich nun zur Aufgabe gestellt, weitere Wirkstoffe zu finden, welche die NF-κB -Aktivierung hemmen.

Überraschenderweise wurde nun gefunden, daß andere, nicht zu CoA Thioestern metabolisierende R-Arylpropionsäuren über die spezifische Hemmung von Schritten innerhalb der NF-κB-Aktivierungskaskade in das Erkrankungsgeschehen eingreifen können. Wegen der ubiquitären Funktion des Transkriptionsfaktors NF-κB bei der Genregulation sind Arzneimittel mit solchen R-Arylpropionsäuren oder deren Derivaten nicht nur zur bekannten Schmerzlinderung über die antinozizeptive Wirkung am zentralen Nervensystem [DE 40 28 906 C2] geeignet, sondern bei geeigneter Anwendung und Dosis auch bei allen Erkrankungen einsetzbar, bei denen eine Hemmung der NF-κB-Aktivierung therapeutisch vorteilhaft genutzt werden kann. Erfindungsgemäß können diese Arzneimittel somit nicht nur bei Rheuma, sondern auch gemäß Anspruch 1 bei Asthma, Schock, entzündlichen Darmerkrankungen wie Morbus Crohn und Colitis ulcerosa), Strahlenschäden, Arteriosklerose und bei der Behandlung von Abstoßungsreaktionen nach Gewebe- oder Organtransplantationen in jeweils angepaßter Dosis und pharmazeutischer Formulierung eingesetzt werden.

Die hier berichtete Beobachtung der Hemmung der NF-κB-Bildung ist überraschend, weil nach dem Stand der Technik die pharmakologischen Effekte der Arylpropionsäuren anderen Mechanismen zugeschrieben wurden. Dies hat bisher zur Anwendung der Racemate oder der S-Enantiomeren bei Schmerzen oder Entzündungen, in geringerer Dosis, geführt.

In der WO 98/09603 ist weiterhin eine Anwendbarkeit von R-NSAID's bei neoplastischen Erkrankungen, insbesondere Dickdarm- und Brustkrebs, cystischer Fibrose und Alzheimerscher Krankheit beschrieben.

Überraschenderweise wurde nun gefunden, daß R-Flurbiprofen und andere nicht zu CoA Thioestern metabolisierende R-Arylpropionsäuren die NF-κB Aktivierung ca. 100 mal potenter hemmen als die entsprechenden S-Enantiomere. Um eine ausreichende Wirkung zu erzielen, müssen sie jedoch in höheren Dosierungen eingesetzt werden als sie bei der bekannten therapeutischen Anwendung von racemischen Arylpropionsäuren üblich sind. Wegen der guten Verträglichkeit auf Grund der praktisch fehlenden Wirkung dieser R-Arylpropionsäure-Dosierungen auf die periphere Prostaglandinbiosynthese ist es jedoch möglich, bei Anwendung der R-Enantiomere die Dosis so hoch festzulegen, daß die erwünschte Hemmwirkung auf die NF-κB-Aktivierung erzielt wird, ohne daß die von der S-Form herrührenden unerwünschten Wirkungen befürchtet werden müssen. Die Wirkstoffe werden daher vorzugsweise überwiegend frei von S-Enantiomeren, d.h. mit einer optischen Reinheit von über 90 %, insbesondere über 99 %, eingesetzt, falls nicht als "Nebenwirkung" auch die bekannte schmerz- und entzündungshemmende Wirkung des S-Enantiomeren erwünscht ist. Im Gegensatz zu R-Ibuprofen sind diesbezügliche unerwünschte Wirkungen wegen der fehlenden R => S-Inversion bei den nicht zu CoA Thioester metabolisierenden R-Arylpropionsäuren nicht zu erwarten. Die erfindungsgemäßen Arzneimittel lassen somit eine verbesserte therapeutische Breite im Vergleich zur Anwendung der racemischen Arylpropionsäuren bzw. deren S-Enantiomere erwarten. Die durchgeführten Untersuchungen am Menschen belegen die gute gastrointestinale Verträglichkeit von R-Flurbiprofen und anderen R-Arylpropionsäuren [Jerussi et al.: Clinical endoscopic evaluation of the gastroduodenal tolerance to Ketoprofen, Flurbiprofen, Racemic Ketoprofen, and Paracetamol: A randomized, single-blind, placebo-controlled trial; J Clin Pharmacol, 38, 19S-24S (1998)], die sich schon in früher durchgeführten Tierexperimenten angedeutet hat [DE 40 28 906 C2].

Seit der Entdeckung des nukleären Transkriptionsfaktors NF-κB vor etwa einem Jahrzehnt werden umfangreiche Forschungsarbeiten zur biologischen Funktion und zur Beeinflussung der NF-κB-Bildung durch endogene und exogene Substanzen durchgeführt. Von den bekannten pharmakologischen Substanzen wurden bisher u.a. Glukocortikoide wie Dexamethason und Prednison, Immunsuppressiva wie Cyclosporin, Tacrolimus und Deoxyspergualin bei therapeutischen Konzentrationen als wirksam auf die NF-κB-Aktivierung beschrieben. Für den bei der biochemischen Inversion von R-Ibuprofen zu S-Ibuprofen intermediär entstehenden Metaboliten, einen R-Ibuprofen-Coenzym A-thioester wurde ebenfalls eine Hemmung der NF-κB-Aktivierung nachgewiesen und spekulativ angenommen, daß auch R-Ibuprofen über die bekannte metabolische Aktivierung im menschlichen Körper zum R-Ibuprofen-CoA-thioester eine Wirkung aufweisen würde, die R-Ibuprofen selbst nicht besitzt. [Brune et al.: Arzneimittel, enthaltend Ibuprofenthioester als Hemmer der Nf-κB abhängigen Bildung von Mediatoren von Entzündungen und Schmerz, DE 197 16 713 A1, WO 98/47502].

Überraschenderweise wurde nun gefunden, daß die therapeutisch genutzten Arylpropionsäurederivate, Flurbiprofen, Ketoprofen, Naproxen und Tiaprofensäure die keine merkliche Bildung von CoA Thioestern beim Menschen aufweisen eine ausgeprägte Hemmung der Aktivierung von NF-κB bewirken und somit das Potential für die mit der Beeinflussung dieses Mechanismus zusammenhängenden therapeutischen Effekte besitzen.

Die erfindungsgemäßen Arzneimittel auf der Basis von R-Arylpropionsäuren und deren Derivaten gemäß Anspruch 1 als Hemmer der NF-κB-Aktivierung zur Therapie von Erkrankungen, welche durch die Modifizierung der NF-κB-Aktiierung beeinflußt werden, beruht auf folgenden experimentellen Untersuchungen:

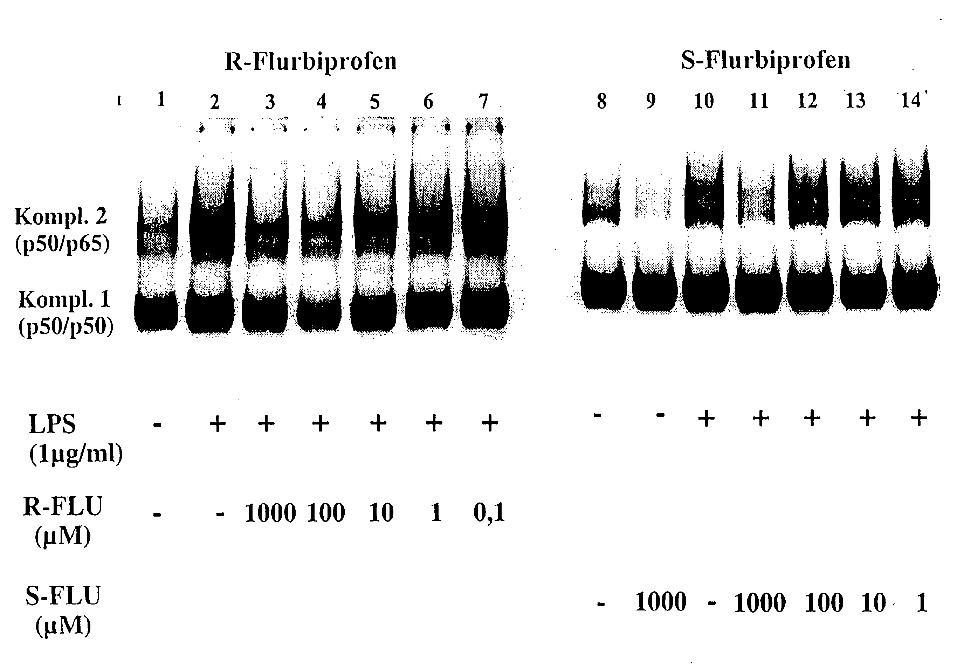

- **Abb. 2**:: Konzentrationsabhängiger Einfluß von R- und S-Flurbiprofen auf die Aktivierung des Transkriptionsfaktors NF-κB in RAW-Zellen. Die Gelretentionsanalyse (Electro Mobility-Shift Assay; DIG Gel Shift Kit, Boehringer Mannheim) zeigt, daß LPS (1 µg/ml) zu einer Aktivierung von NF-κB (p50/p65 Komplex von NF-κB) führt (Spur Nr. 2 und 10). Mikromolare Konzentrationen von R-Flurbiprofen (Spur Nr. 3, 4, 5, 6, 7 gegen Spur Nr. 2 als Kontrolle) waren in der Lage diese LPS induzierte Aktivierung von NF-κB zu hemmen. Eine densitometrische Auswertung ergab, daß S-Flurbiprofen hinsichtlich dieser Eigenschaften ca. 100 mal weniger potent war (Spur Nr. 11, 12, 13,14, gegen Spur Nr. 10 als Kontrolle). Spur Nr. 1 und 8 zeigen jeweils unstimulierte Kontrollzellen.

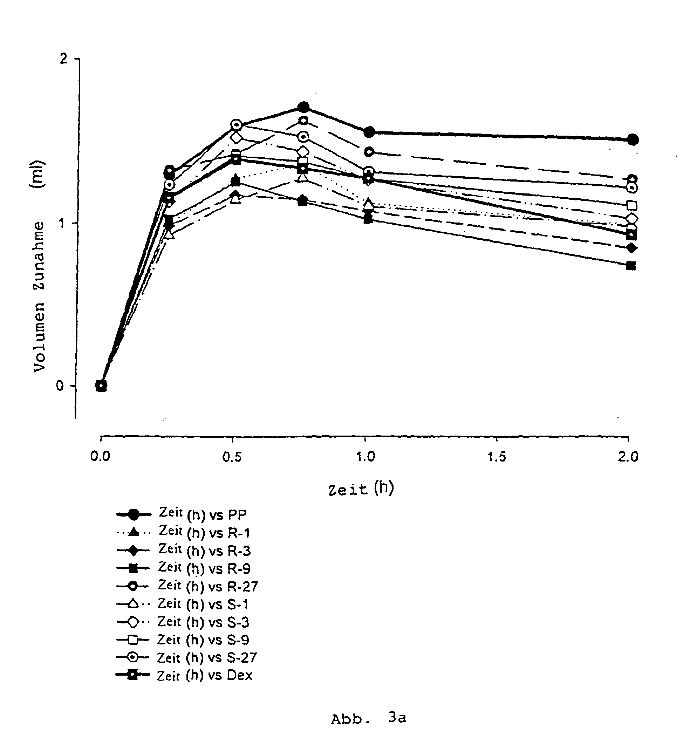

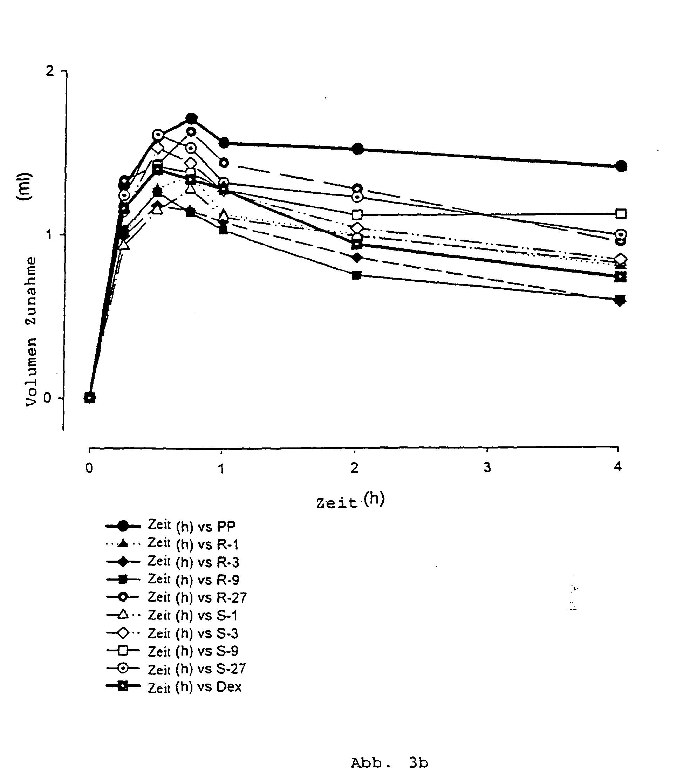

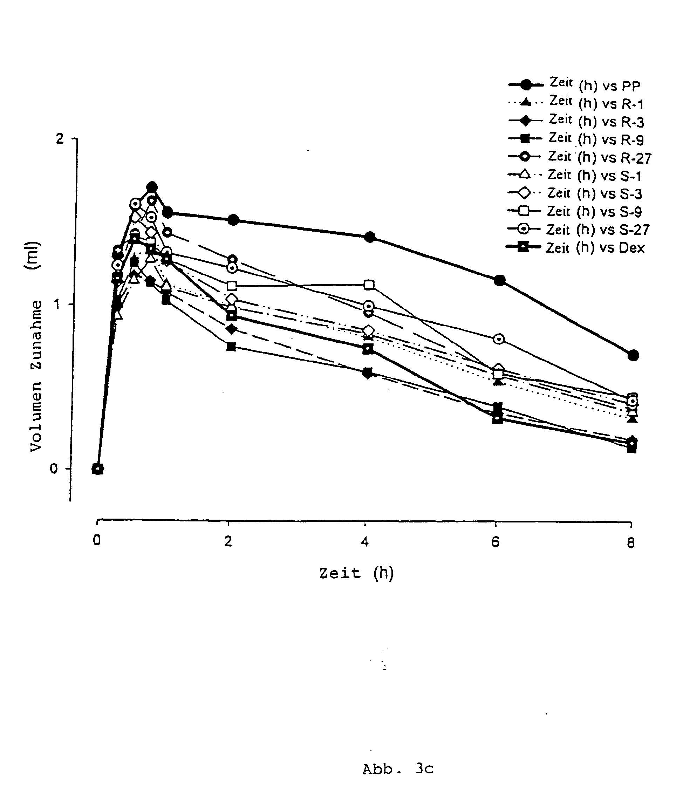

Da der nukleäre Transkriptionsfaktor NF-κB u.a. für die Bildung, einer Reihe von Enzymen mit proinflammatorischer und ödembildender Eigenschaften verantwortlich ist, wurde der Einfluß von R-Flurbiprofen auf das Zymosan-induzierte Rattenpfotenödem bestimmt (Methode beschrieben bei: Meller ST und Gebhart GF: Intraplantar zymosan as a reliable, quantifiable model of thermal and mechanical hyperalgesia in the rat; European Journal of Pain, 1, 43-52 (1997). **Abbildung 3 a - c** faßt die Ergebnisse zusammen.
- **Abb. 3a - c:**: Zeitabhängige Zunahme des Rattenpfotenvolumens (gemessen mit einem Plethysmographen) nach intraplantarer Applikation von Zymosan. Nach Applikation von Zymosan [Meller and Gebhart (s.o.)] in eine Hinterpfote der Ratte kommt es als Zeichen einer Entzündung zur Zunahme des Pfotenvolumens (Placebo-Gruppe, Applikation von Vehicle = Phosphatpuffer (PP)). Aufgrund der
hemmenden Wirkung von R-Flurbiprofen auf die NF-κB Aktivierung ist bei Dosierungen im Bereich zwischen 1 und 27 mg/kg Körpergewicht (Applikation: intraperitoneal) eine überraschende Abnahme des Pfotenvolumens ersichtlich. Dieser Effekt war besonders ausgeprägt zwischen der 2. und 6. Stunde nach Zymosan-Applikation. Dexamethason (0,5 mg/kg Körpergewicht), ein bekannter Hemmer der NF-κB Aktivierung, wurde als Positivkontrolle eingesetzt. S-Flurbiprofen zeigte erwartungsgemäß ebenfalls eine Reduktion des Pfotenvolumens, wobei dieser Effekt jedoch nicht über eine Hemmung der NF-κB Aktivierung, sondern über eine Hemmung der Synthese von proinflammatorischen Prostaglandinen erklärbar ist. S-Flurbiprofen ist ein bekannter Hemmer der Cyclooxygenasen.

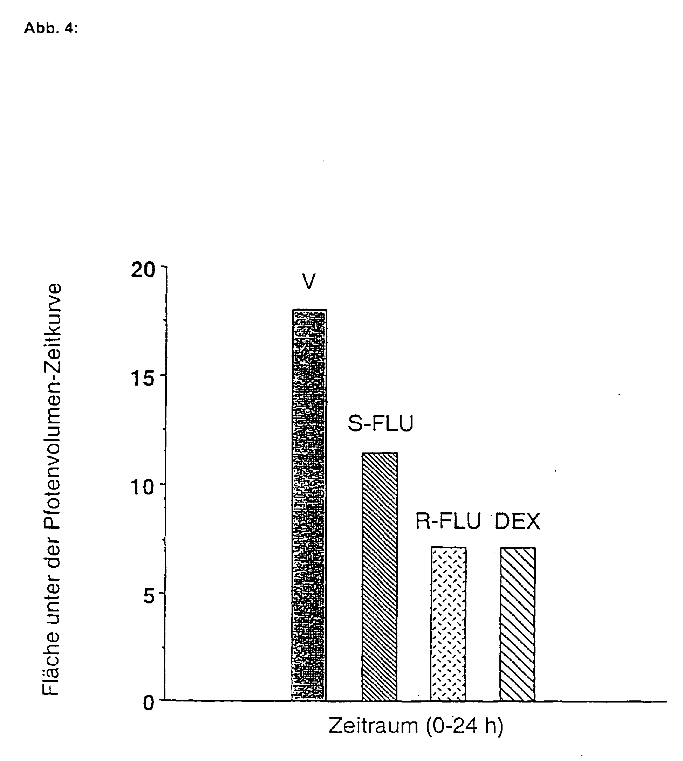

- **Abb. 4**:: Zusammenfassung der Effekte von 9 mg/kg R-Flurbiprofen, 9 mg/kg S-Flurbiprofen und 0,5 mg/kg Dexamethason gegen Placebo (V) über 24 Stunden. Die Effekte nach 9 mg/kg R-Flurbiprofen waren vergleichbar mit denen nach 0,5 mg/kg Dexamethason.

Die Herstellung und chirale Trennung der Arylpropionsäuren und ihrer Derivate ist bekannt. Beispielhaft sei auf die WO 93-17677 und die darin genannte Literatur verwiesen.

Unter Arylpropionsäurederivaten werden erfindungsgemäß die im Magen/Darmtrakt (bei oraler Applikation) bzw. im Blut in die Arylpropionsäuren rückgespaltenen Derivate verstanden, wie Alkylester mit 1-6 C-Atomen, die ggf. Amino- oder Hydroxygruppen enthalten können, Amide oder Alkylamide mit 1-6 C-Atomen, sowie die pharmazeutisch verträglichen Salze, insbesondere Alkali-, Erdalkali-, Ammonium-, Aminosäuresalz, vorzugsweise Lysinat, Megluminat, Trometamin, Arginat oder Aluminiumsalz. Auch solche Verbindungen sind bekannt.

Die Bedeutung einer prophylaktischen oder therapeutischen Gabe von R-Arylpropionsäure in der akuten oder chronischen Behandlung von Krankheiten wird entsprechend der Stärke der zu behandelnden Beschwerden variieren. Die Dosis und die Häufigkeit der Dosierungen werden sich ebenfalls entsprechend dem Alter, Körpergewicht und Reaktion des einzelnen Patienten unterscheiden. Generell sollte die Tagesdosis von R-Arylpropionsäure für die vorliegend beschriebenen Beschwerden zwischen ca. 200mg und ca. 2000 mg liegen, verabreicht in einer oder mehreren Gaben. Vorzugsweise liegt die Tagesdosis zwischen ca. 200 mg und ca. 500 mg, verabreicht in einer oder mehreren Gaben. Bei der Betreuung des Patienten sollte die Behandlung mit einer niederen Dosierung begonnen werden, möglicherweise von 200 mg und bis zu ca. 1000 mg oder höher gesteigert werden, je nach der allgemeinen Reaktion des Patienten. Es wird ferner empfohlen, daß Säuglinge, Kinder, Patienten über 65 Jahre und solche mit beeinträchtigter Nieren- oder Leberfunktion zuerst eine niedere Dosis erhalten und basierend auf der individuellen Reaktion und dem Blutspiegel titriert werden. In manchen Fällen kann es erforderlich sein, eine Dosierung außerhalb dieser Bereiche anzuwenden, was für den Fachmann offensichtlich ist. Weiterhin wird angemerkt, daß der behandelnde Hausarzt oder klinische Facharzt im Zusammenhang mit der allgemeinen Reaktion des Patienten weiß, wie und wann die Behandlung zu unterbrechen, umzustellen oder abzubrechen ist. Die Ausdrucksweise "eine Menge, welche für die NF-κB-Hemmung ausreichend ist, aber nicht ausreicht, um nachteilige Reaktionen (Prostaglandinsynthesehemmung) auszulösen" ist von den oben angegebenen Dosierungsmengen und der Dosierungsanleitung umfaßt. Jede beliebige Art der Verabreichung kann angewendet werden, um den Patienten mit einer wirksamen Dosierung der R-Arylpropionsäure zu versorgen. Beispielsweise oral, rektal, transdermal, parenteral (subkutan, intramuskulär, intravenös), intrathekal, epi- oder peridural und ähnliche Formen der Verabreichung können angewendet werden. Mögliche Applikationsformen sind z.B. Tabletten, Dispersionen, Suspensionen, Lösungen, Kapseln, Pflaster und dergleichen.

Die pharmazeutischen Formulierungen umfassen R-Arylpropionsäure als Wirkstoff oder ein pharmazeutisch verträgliches Derivat davon und einen pharmazeutisch verträglichen Trägerstoff und wahlweise andere therapeutische Zusätze.

Die Ausdrucksweisen "pharmazeutisch verträgliche Derivate" oder "ein pharmazeutisch verträgliches Derivat davon" beziehen sich auf Derivate hergestellt aus pharmazeutisch verträglichen, nichttoxischen Säuren oder Basen, einschließlich anorganischer Säuren und Basen und organischer Säuren und Basen. Da die Komponente der vorliegenden Erfindung sauer ist, können Derivate mit pharmazeutisch verträglichen, nichttoxischen Basen, einschließlich anorganischer und organischer Basen, hergestellt werden. Geeignete pharmazeutisch verträgliche basische Zusatzderivate für die Komponente der vorliegenden Erfindung umfassen Metallsalze, hergestellt aus Aluminium, Calcium, Lithium, Magnesium, Kalium, Natrium und Zink oder organische Salze hergestellt aus Lysin, N,N'-Dibenzylethylendiamin, Cholin, Diethanolamin, Ethylendiamin, Meglumin (N-methylglucamin), Trometamin, Arginin und Alkylaminen mit 1-6 C-Atomen.

Die Formulierungen umfassen Formulierungen wie Suspensionen, Lösungen, Elixiere und Aerosole. Trägerstoffe wie Stärke, Zucker, mikrokristalline Zellulose, Verdünner, Granulierhilfsmittel, Gleitmittel, Bindemittel, Lösemittel und ähnliches können im Falle der festen oralen Applikationsformen verwendet werden. Feste orale Applikationsformen (wie Pulver, Kapseln und Tabletten) werden den flüssigen oralen Applikationsformen vorgezogen. Die bevorzugte feste orale Applikationsform sind Tabletten. Die Tabletten können auf Wunsch mit standardisierten Wasser- oder wasserfreien Beschichtungsmitteln überzogen werden.

Zusätzlich zu den üblichen oben angeführten Applikationsformen kann die erfindungsgemäße Komponente mit an sich bekannten Mitteln in verzögert anflutender und/oder rasch anflutender Form verabreicht werden. Beispielsweise wirken hydrophobierende Zusätze zu oralen Applikationsformen verzögernd, Sprengmittel und Tenside auflösungsfördernd und damit beschleunigend und, wie bekannt, können beide Formen in Granulatform gemischt werden, um einen Teil des Wirkstoffs rasch und den Rest verzögert anfluten lassen.

Pharmazeutische Formulierungen, welche für die orale Applikationsform geeignet sind, können als separate Einheiten wie Kapseln, Dragees oder Tabletten, oder Aerosole, jeweils eine vorgegebene Menge des Wirkstoffes in Form von Pulver- oder Granulat, oder als Lösung oder Suspension in einer wäßrigen Flüssigkeit, einer nicht wäßrigen Flüssigkeit, einer Öl-in-Wasser Emulsion oder einer flüssigen Wasser-in-Öl Emulsion enthalten. Solche Formulierungen können nach jeder pharmazeutischen Methode hergestellt werden, aber alle Methoden beinhalten eine Vermischung des Wirkstoffes mit einer Trägersubstanz, welche aus einem oder mehreren der notwendigen Bestandteile besteht. Generell werden die Formulierungen durch gleichmäßiges und gründliches Vermischen des Wirkstoffes mit flüssigen Trägersubstanzen oder feinzerkleinerten festen Trägersubstanzen, oder beidem, und dann, falls erforderlich, Formen des Produktes in die gewünschte Applikationsform, hergestellt.

Beispielsweise kann eine Tablette durch Pressen oder Formen, wahlweise mit einem oder mehreren zusätzlichen Bestandteilen hergestellt werden. Gepreßte Tabletten können durch Verpressen in einer entsprechenden Vorrichtung hergestellt werden, wenn der Wirkstoff in einer rieselfähigen Form wie Pulver oder Granulat, wahlweise gemischt mit einem Bindemittel, Gleitmittel, inerten Verdünner, Dispergier- oder oberflächenaktiven Mittel, vorliegt. Geformte Tabletten können auch durch Formen einer Mischung der pulverisierten Komponenten, befeuchtet mit einem inerten flüssigen Verdünner, in einer geeigneten Vorrichtung und anschließender Trocknung hergestellt werden. Vorzugsweise enthält jede Tablette zwischen 200 mg und 1000 mg des Wirkstoffes, und jedes Dragee oder Kapsel enthält zwischen ca. 200 mg und ca. 600 mg des Wirkstoffes. Besonders bevorzugt enthält die Tablette, Dragee oder Kapsel eine von zwei Dosierungen, nämlich 200 mg oder 500 mg des Wirkstoffes.

## Patentansprüche

1. Verwendung der R-Arylpropionsäuren R-Flurbiprofen, R-Ketoprofen, R-Naproxen und/oder R-Tiaprofensäure oder ihrer Derivate, zur Herstellung von humanen Arzneimitteln, zur Bekämpfung von Krankheiten durch die Hemmung der NF-κB-Aktivierungskaskade ausgewählt aus der Gruppe von rheumatischen Erkrankungen, Asthma, Schock, entzündliche Darmerkrankungen wie Morbus Crohn und Colitis ulcerosa, Strahlenschäden, Arteriosklerose und Abstoßungsreaktionen nach Gewebe- oder Organtransplantationen, wobei die Mittel die R-aryl propionsäuren in einer Menge von 200 - 1000 mg/ Dosis enthalten.

2. Verwendung nach Anspruch 1 **dadurch gekennzeichnet, dass** die Mittel die R-Arylpropionssäure in einer Menge von 200-500 mg/Dosis enthalten.

3. Verwendung nach Anspruch 1 oder 2 **dadurch gekennzeichnet, dass** die R-Arylpropionsäuren oder R-Arylpropionsäurederivate weniger als 1 % S-Arylpropionsäuren oder S-Arylpropionsäurederivate enthalten.

4. Verwendung nach Anspruch 1 bis 3 **dadurch gekennzeichnet, dass** die Wirkstoffe als Alkali-, Erdalkali-, Ammonium-, Aminosäuresalz, vorzugsweise als Lysinat, Megluminat, Trometamin, Arginat oder als.Aluminiumsalz vorliegen.

5. Verwendung nach Anspruch 1 bis 4 **dadurch gekennzeichnet, dass** die Arzneimittel übliche Hilfs- und Trägerstoffe enthalten.

## Claims

1. Use of the R-arylpropionic acids R-flurbiprofen, R-ketoprofen, R-naproxen and/or R-tiaprofenic acid or their derivatives for the preparation of human medicaments for the combatting of diseases influenced by the inhibition of the NF-kB activation cascade selected from the group of rheumatic diseases, asthma, shock, inflammatory intestinal diseases like Crohn's disease and colitis ulcerosa, radiation damages, arteriosclerosis and rejection reactions after tissue and organ transplants whereby the medicaments contain the P-arylpropionic acids in an amount of 200-1000 mg/dose.

2. Use according to claim 1, **characterised in that** the medicament contains the R-arylpropionic acid in an amount of 200-500 mg/dose.

3. Use according to claim 1 or 2, **characterised in that** the R-arylpropionic acid or R-arylpropionic acid derivatives contains less than 1 % of S-arylpropionic acids or S-arylpropionic acid derivatives.

4. Use according to claim 1 to 3, **characterised in that** the active material is present as alkali metal, alkaline earth metal, ammonium, amino acid salt, preferably as lysinate, megluminate, trometamine, arginate or aluminium salt.

5. Use according to claim 1 to 4, **characterised in that** the medicament contains usual adjuvant and carrier materials.

## Revendications

1. Utilisation des acides R-arylpropioniques R-flurbiprofène, R-kétoprofène, R-naproxène et/ou de l'acide R-thiaprofénique ou de leurs dérivés pour la préparation de médicaments humains destinés au traitement, par inhibition de la cascade d'activation du facteur de transcription NF-κB, de maladies du groupe comprenant les maladies rhumatismales, l'asthme, le choc, les maladies inflammatoires de l'intestin telles que la maladie de Crohn et la colite ulcéreuse, les lésions d'irradiation, l'artériosclérose et les réactions de rejet après transplantation de tissus ou d'organes, le médicament comprenant l'acide R-arylpropionique dans une proportion de 200-1000 mg/dose.

2. Utilisation selon la revendication 1, **caractérisée en ce que** l'agent contient l'acide R-arylpropionique dans une proportion de 200-500 mg/dose.

3. Utilisation selon la revendication 1 ou 2, **caractérisée en ce que** les acides R-arylpropioniques ou les dérivés d'acides R-arylpropioniques contiennent moins de 1 % d'acides S-arylpropioniques ou de dérivés d'acides S-arylpropioniques.

4. Utilisation selon l'une quelconque des revendications 1 à 3, **caractérisée en ce que** les principes actifs sont utilisés sous forme de sels de métaux alcalins, de métaux alcalino-terreux, d'ammonium, d'acides aminés, de préférence sous forme de lysinate, de mégluminate, de trométamine, d'arginate ou sous forme de sel d'aluminium.

5. Utilisation selon l'une quelconque des revendications 1 à 4, **caractérisée en ce que** le médicament contient des adjuvants et véhicules usuels.
